# EUROPÄISCHE PATENTANMELDUNG

(11) **EP 2 042 096 A1**
(43) Veröffentlichungstag der Anmeldung: **01.04.2009**
(21) Anmeldenummer: 07019012.9
(22) Anmeldetag: 27.09.2007
(51) Int. Cl.: A61B 5/15, A61M 39/22, G01N 35/10, F16K 11/085

(54) **Verteilvorrichtung für eine Körperflüssigkeitsprobe, Fluidentnahme- und infusionssystem sowie Verfahren zum Betreiben**

(71) Anmelder: F. Hoffmann-La Roche AG, 4070 Basel (CH)
(72) Erfinder: Schweiger, Gerd, 8010 Graz (AT)
(74) Vertreter: Bittner, Thomas L.

(57) **Zusammenfassung**

Die Erfindung bezieht sich auf eine Verteilvorrichtung (1) für eine Körperflüssigkeitsprobe, insbesondere eine Blutprobe, mit einem äußeren Bauteil (13), an welchem mehrere Anschlüsse gebildet sind, und einem inneren Bauteil (12), welches wenigstens teilweise in einer Aufnahme in dem äußeren Bauteil (13) angeordnet ist, zwischen Verlagerungsstellungen verlagerbar ist und einen zum Aufnehmen einer Fluidprobe konfigurierten Hohlraum (11) aufweist, wobei in einer ersten Stellung des inneren Bauteils (12) über den Hohlraum (11) zwischen einem ersten Anschluss (4) und einem ersten zugeordneten Anschluss (10), welcher dem ersten Anschluss (4) zugeordnet ist, eine bidirektionale Fluidverbindung gebildet ist und der Hohlraum (11) getrennt von einem zweiten Anschluss (7) ist, und in einer zweiten Stellung der Hohlraum (11) von dem ersten Anschluss (4) und dem ersten zugeordneten Anschluss (10) getrennt ist und in Fluidverbindung mit dem zweiten Anschluss (7) steht. Die Erfindung bezieht sich ferner auf ein Fluidentnahme- und Infusionssystem sowie ein Verfahren zum Betreiben derselben.

## Beschreibung

Die Erfindung bezieht sich auf eine Verteilvorrichtung für eine Körperflüssigkeitsprobe, insbesondere eine Blutprobe, ein Fluidentnahme- und Infusionssystem sowie ein Verfahren zum Betreiben derselben.

### Hintergrund der Erfindung

Fluidentnahmesysteme werden beispielsweise auf dem Gebiet der Medizin-, der Labor- oder der Analysetechnik verwendet. Zum Beispiel findet eine Anwendung in Systemen statt, die der Entnahme einer Probe einer Körperflüssigkeit sowie einer anschließend wahlweise ausgeführten Analyse der entnommenen Probe in einem hierfür geeigneten Analysegerät dienen. Insbesondere werden solche Systeme dafür genutzt, einem Patienten. Blut zu entnehmen und wenigstens einen Teil des entnommenen Blutes in einem Blutanalysegerät zu untersuchen. In diesem Zusammenhang kann auch vorgesehen sein, dass die Blutentnahme kontinuierlich automatisiert erfolgt. Die Entnahme kann sowohl für arterielles als auch venöses Blut erfolgen. Eine anschließende Blutanalyse erfolgt, um handlungsrelevante Informationen über den Zustand des Patienten zu gewinnen, und ermöglicht daher eine gezielte Betreuung und Therapie. Mit Hilfe von in geeigneter Weise ausgestalteten Analysegeräten können verschiedene Blutparameter untersucht werden, zu denen beispielsweise der Partialdruck von Sauerstoff und Kohlendioxid als auch die Sauerstoffsättigung gehören. Andere Parameter sind der pH-Wert, der Hämatokritwert oder der Gehalt an Natrium, Kalzium oder Kalium.

Kombiniert werden derartige Systeme zur Entnahme einer Probe der Körperflüssigkeit, beispielsweise einer Blutprobe, häufig mit Systemkomponenten für eine Infusion, wobei die Infusion gekoppelt an das Ergebnis der Analyse der entnommenen Körperflüssigkeitsprobe oder losgelöst hiervon ausführbar ist. In einem solchen System kann beispielsweise eine in Fluidleitungen integrierte Komponente wie ein 3-Wegehahn zwischen Patient und genutzten Geräten vorgesehen sein, insbesondere dem Analysegerät und einer Infusionsvorrichtung. Mit der Komponente wird Einfluss auf den Strom der Flüssigkeiten in den Fluidleitungen genommen, die Patient und angeschlossene Geräte bedarfsweise verbinden.

In Verbindung mit einem Entnahme- und Infusionssystem besteht häufig die Anforderung, dem Patienten die Infusionslösung mit variabler Flussrate über eine Leitung zuzuführen. Mittels Umkehr der Flussrichtung in der Leitung kann dem Patienten Blut entnommen werden. Eine Verteilvorrichtung in der Leitung dient dann dazu, eine Blutprobe bereitzustellen, die anschließend mit Hilfe eines Analysegerätes untersucht werden kann. Nach der Probennahme wird folgend auf eine erneute Flussrichtungsumkehr die Infusion fortgesetzt. Hierbei erfolgt gleichzeitig eine Säuberung der Fluidleitung von Blutresten.

Bei Vorrichtungen für die Entnahme einer Probe einer Körperflüssigkeit ist die Verwendung eines Septums bekannt. Hierbei wird die Probe aus der Fluidleitung unter Verwendung einer Kapillare oder einer Nadel entnommen, indem das Septum mittels der Kapillare oder der Nadel durchstochen wird, so dass die Kapillarenspitze oder die Nadelspitze in der Leitung in Kotakt mit der Körperflüssigkeit kommt. Nach der Entnahme durch die Kapillare oder die Nadel wird diese wieder aus dem Septum herausgezogen. Zur Wahrung der Sterilität muss die Kapillare oder die Nadel entweder vor jeder Entnahme desinfiziert werden, oder es wird für jede Entnahme ein neuer Einwegartikel genutzt. Hierbei treten Probleme dahingehend auf, dass die Außenseite des Septum auch zwischen zwei Probenentnahmen gegen Kontamination geschützt werden muss.

Es ist weiterhin bekannt, eine Körperflüssigkeitsprobe aus einer Fluidleitung zu entnehmen, indem die Probe durch ein Überdruckventil aus der Leitung abgegeben wird. Die so entnommene Probe kann dann dem gewünschten Analysegerät zugeführt werden. Optional kann zusätzlich ein 3-Wegehahn eingesetzt werden. Während das Überdruckventil geöffnet ist, besteht bei diesem System eine direkte Fluidverbindung zwischen der Zuleitung zu dem Analysegerät und dem Patienten. Dieses stellt ein inhärentes Sicherheitsrisiko dar. Weiterhin sind Probleme bei der Ventildichtung im Betrieb beobachtet worden, so dass es schwierig ist, hohe Anforderungen an die mit dem Überdruckventil gebildete sterile Barriere zwischen Analysegerät und Patient zu erfüllen. Aus dem Dokument US 4,608,996 ist ein 3-Wegehahn für Fluide mit drei Stellungen bekannt. In jeder der drei Stellungen ist eine Fluidverbindung zwischen genau zwei von drei äußeren Anschlüssen gebildet, nämlich einem Probenanschluss, einen Patientenanschluss und einem Ausgangsanschluss.

Aus dem Dokument US 5,758,643 sind ein Verfahren und eine Vorrichtung zum Überwachen der Blutchemie bekannt. Das System ermöglicht die Überwachung der Blutgase und Elektrolyte. Es kann Blut aus dem Kreislauf des Patienten mittels Katheter und Pumpe entnehmen. Der Aufbau der Analyseeinheit beinhaltet mehrere Sensoren, um die verschiedenen Parameter zu bestimmen. Diese Einheit befindet sich in unmittelbarer Nähe des Patientenkatheters. Nach der Analyse wird der Rest des abgenommenen Blutes wieder reinfundiert und das System mit Infusionsflüssigkeit gespült. Eine interne Überwachung ermöglicht es, den Patienten mit dem Analysator, mittels Anschlusssystemen unterschiedlicher Länge und unterschiedlichem Volumen zu verbinden.

Weiterhin ist aus dem Dokument US 5,165,406 eine Sensoranordnung für ein kombiniertes Infusions- und Blutanalysesystem bekannt. Eine Sensoranordnung weist eine Elektrodenanordnung auf, die in einem Elektrodenraum montiert ist.

### Zusammenfassung der Erfindung

Aufgabe der Erfindung ist es, eine Verteilvorrichtung für eine Körperflüssigkeitsprobe, insbesondere eine Blutprobe, sowie ein Fluidentnahme- und Infusionssystem und ein Verfahren zum Betreiben der Verteilvorrichtung zu schaffen, bei denen die Körperflüssigkeitsprobe auch bei häufiger Nutzung zuverlässig und je nach Anwendungszweck in gewünschter Art und Weise sicher entnommen oder verteilt werden kann. Hierbei soll des weiteren die Einhaltung hoher Sterilitätsanforderungen ermöglicht sein.

Die Erfindung umfasst den Gedanken einer Verteilvorrichtung für eine Körperflüssigkeitsprobe, insbesondere eine Blutprobe, mit einem äußeren Bauteil, an welchem mehrere Anschlüsse gebildet sind, und einem inneren Bauteil, welches wenigstens teilweise in einer Aufnahme in dem äußeren Bauteil angeordnet ist, zwischen Verlagerungsstellungen verlagerbar ist und einen zum Aufnehmen einer Körperflüssigkeitsprobe konfigurierten Hohlraum aufweist, wobei in einer ersten Stellung des inneren Bauteils über den Hohlraum zwischen einem ersten Anschluss und einem ersten zugeordneten Anschluss, welcher dem ersten Anschluss zugeordnet ist, eine bidirektionale Fluidverbindung gebildet ist und der Hohlraum getrennt von einem zweiten Anschluss ist, und in einer zweiten Stellung der Hohlraum von dem ersten Anschluss und dem ersten zugeordneten Anschluss getrennt ist und in Fluidverbindung mit dem zweiten Anschluss steht.

Unter Verwendung wenigstens einer solchen Verteilvorrichtung ist nach einem weiteren Aspekt der Erfindung ein Körperflüssigkeitsentnahme- und Infusionssystem, insbesondere Blutentnahme- und Infusionssystem, mit einer Probenverteilvorrichtung geschaffen, bei der ein patientenseitiger Anschluss, ein infusionsseitiger Anschluss und ein analysatorseitiger Anschluss gebildet sind. Über den patientenseitigen Anschluss gelangt eine Körperflüssigkeit des Patienten, insbesondere Blut, zu der Probenverteilvorrichtung, nämlich in den Hohlraum. Wenn das System zur Infusion genutzt wird, dient der patientenseitige Anschluss weiterhin dazu, eine über den infusionsseitigen Anschluss zugeführte Infusionsflüssigkeit in eine Leitung zu dem Patienten einzuspeisen. Der analysatorseitige Anschluss dient zur Abgabe einer entnommenen Probe an ein Analysegerät, bedarfsweise über ein geeignetes Leitungssystem.

Nach einem weiteren Aspekt der Erfindung ist ein Verfahren zum Betreiben einer Verteilvorrichtung für eine Körperflüssigkeitsprobe in einem Körperflüssigkeitsentnahme- und Infusionssystem, insbesondere einem Blutentnahme- und Infusionssystem, mit einem äußeren Bauteil, an welchem mehrere Anschlüsse gebildet sind, und einem inneren Bauteil geschaffen, welches wenigstens teilweise in einer Aufnahme in dem äußeren Bauteil angeordnet ist, zwischen Verlagerungsstellungen verlagerbar ist und einen zum Aufnehmen einer Körperflüssigkeitsprobe konfigurierten Hohlraum aufweist, wobei das Verfahren die folgenden Schritte aufweist:
- Anordnen des inneren Bauteils in einer ersten Stellung, in welcher über den Hohlraum zwischen einem ersten Anschluss und einem ersten zugeordneten Anschluss, welcher dem ersten Anschluss zugeordnet ist, eine bidirektionale Fluidverbindung gebildet und der Hohlraum getrennt von einem zweiten Anschluss ist,
- Einbringen einer Körperflüssigkeitsprobe über den ersten Anschluss in den Hohlraum,
- Verlagern des inneren Bauteils in eine zweite Stellung, in welcher der Hohlraum von dem ersten Anschluss und dem ersten zugeordneten Anschluss getrennt ist und in Fluidverbindung mit dem zweiten Anschluss steht,
- wenigstens teilweises Abgeben der Körperflüssigkeitsprobe aus dem inneren Hohlraum über den zweiten Anschluss,
- Verlagern des inneren Bauteils in die erste Stellung und
- Bereitstellen einer Infusionsflüssigkeit an dem ersten zugeordneten Anschluss für eine Abgabe über den Hohlraum und den ersten Anschluss.

Das Verfahren kann in einer bevorzugten Ausgestaltung einen Schritt umfassen, welcher als Spülschritt ausgeführt wird, zum Beispiel mittels einer physiologischen Salzlösung oder einer Infusionslösung, und bei dem der Hohlraum, einschließlich der in der gewählten Stellung ankoppelnden Anschlüsse, luftfrei gemacht werden, wodurch Luftblasen vermieden werden. Der Spülschritt kann ausgeführt werden, wenn der Hohlraum in beiden Endbereich jeweils mit einem Anschluss verbunden ist, so dass das Spülfluid durch den Hohlraum hindurch von dem einen zu dem anderen Anschluss fließt. Aber auch ein Einströmen und Ausströmen des Spülfluids durch den gleichen Anschluss kann vorgesehen sein, wenn zum Beispiel der Hohlraum in der zum Spülen gewählten Stellung mit nur einem Anschluss in Verbindung steht.

Darüber hinaus sieht ein Aspekt der Erfindung eine Körperflüssigkeitsanalysevorrichtung, insbesondere Blutanalysevorrichtung, mit einer integrierten Verteilvorrichtung vor, wobei der erste Anschluss als ein patientenseitiger Anschluss, der erste zugeordnete Anschluss als ein infusionsseitiger Anschluss und der zweite Anschluss als ein analysatorseitiger Anschluss gebildet sind.

Mit der vorgeschlagen Verteilvorrichtung für die Körperflüssigkeitsprobe ist es für den Benutzer auf einfache Art und Weise ermöglicht, die Körperflüssigkeitsprobe mittels Einströmen der Körperflüssigkeit in den Hohlraum zu sammeln und anschließend mit Hilfe einer Verlagerung des inneren Bauteils die gesammelte Körperflüssigkeitsprobe ganz oder teilweise über den zweiten Anschluss abzugeben. Ein Durchfluss zwischen dem ersten und dem ersten zugeordneten Anschluss ist mittels der bidirektionalen Fluidverbindung über den Hohlraum ermöglicht, die bei Verwendung in einem Körperflüssigkeitsentnahme- und Infusionssystem je nach Flussrichtung zur Entnahme einer Körperflüssigkeit oder zum Infundieren einer Flüssigkeit nutzbar ist. Beim Infundieren erfolgt nicht notwendigerweise das Einbringen einer Infusionsflüssigkeit zu therapeutischen Zwecken, auch wenn die Verteilvorrichtung ebenfalls in einer solchen Anwendung nutzbar ist. Vielmehr kann eine Flüssigkeit in die Verteilvorrichtung eingebracht werden, um Reste der Körperflüssigkeit in den Körper zurück zu spülen.

Bei der Entnahme der Körperflüssigkeitsprobe, zum Beispiel einer Blutabnahme bei einem Patienten, kann über den ersten zugeordneten Anschluss ein Unterdruck aufgegeben werden, um für die Fluidentnahme einen Sog zu erzeugen, der dazu führt, dass die Körperflüssigkeit in den Hohlraum einströmt. Hohe Sterilitätsanforderungen werden hierbei erfüllt, da weder in der Stellung noch in der weiteren Stellung eine Fluidverbindung zwischen dem ersten Anschluss und dem zweiten Anschluss besteht. In beiden Stellungen steht der Hohlraum, in welchem sich die Körperflüssigkeitsprobe sammelt, lediglich mit einem der beiden Anschlüsse in Fluidverbindung. Ein direkter Fluss zwischen den beiden Anschlüssen ist so wirksam unterbunden, wodurch eine effektive Barriere zwischen den Anschlüssen gebildet ist.

Das innere Bauteil mit dem Hohlraum kann in einer möglichen Ausgestaltung aus einem Vollmaterial hergestellt sein, in welches in einer einfachen Ausführung eine Bohrung für den Hohlraum eingebracht ist. Eine andere Ausführungsform sieht vor, dass das innere Bauteil als Spritzgussbauteil ausgeführt ist, so dass die Hohlraumstruktur wenigstens teilweise durch ein Werkzeug vorgegeben ist, in welches ein Kunststoffmaterial bei der Herstellung eingebracht wird.

Eine bevorzugte Weiterbildung der Erfindung sieht vor, dass mit dem Hohlraum eine Kanalstruktur in dem inneren Bauteil gebildet ist. In einer einfachen Fortbildung handelt es sich bei der Kanalstruktur um einen das innere Bauteil durchsetzenden, geraden Kanal, beispielsweise in Form einer Bohrung. Die Kanalstruktur ist in nahezu beliebiger Weise ausgestaltbar und kann mit einer oder mehrerer Öffnungen gebildet sein, durch welche die Körperflüssigkeitsprobe in den Hohlraum ein- oder ausströmen kann.

Bevorzugt sieht eine Fortbildung der Erfindung vor, dass das innere Bauteil mittels einer Drehbewegung zwischen der ersten Stellung und der zweiten Stellung verlagerbar ist. Ein hierfür geeignetes inneres Bauteil weist beispielsweise im wesentlichen die Form eines Zylinders auf, welcher in einem von dem äußeren Bauteil gebildeten Gehäuse angeordnet ist, zweckmäßig passgenau. In Anlehnung an die Zylinderform des inneren Bauteils kann das äußere Bauteil insgesamt einen runden Querschnitt aufweisen, wobei auf der äußeren Umlauffläche die Anschlüsse gebildet sind.

Eine Weiterbildung der Erfindung kann vorsehen, dass das innere Bauteil in die Aufnahme formschlüssig eingepasst ist. Die formschlüssige Einpassung optimiert den Schutz gegen ein nicht beabsichtigtes Überströmen des Fluids zwischen dem ersten und dem zweiten Anschluss. Hierbei ist das innere Bauteil zweckmäßig so eingepasst, dass die Verlagerung zwischen der Stellung und der weiteren Stellung nur unter Überwindung von Reibungskräften möglich ist, wodurch ein unbeabsichtigtes Verlagern zwischen den Stellungen, beispielsweise aufgrund von Vibrationen des Systems oder durch bloßes Anstoßen, vermieden ist. Auf diese Weise wird die Betriebssicherheit erhöht.

Eine vorteilhafte Ausführungsform der Erfindung sieht vor, dass die erste Stellung und die zweite Stellung benachbarte Verlagerungsstellungen des inneren Bauteils sind. Beispielsweise sind die erste Stellung und die zweite Stellung zwei benachbarte Rastposition des inneren Bauteils in dem äußeren Bauteil. Die verschiedenen Verlagerungsstellungen des inneren Bauteils sind beispielsweise dadurch charakterisiert, dass in ihnen jeweils eine Fluidverbindung zwischen dem Hohlraum und irgendeinem Anschluss besteht, der an dem äußeren Bauteil gebildet ist.

Bei einer vorteilhaften Ausgestaltung der Erfindung kann vorgesehen sein, dass der Hohlraum in Zwischenstellungen, welche von der ersten Stellung und der zweiten Stellung verschieden sind, als anschlussfreier Hohlraum gebildet ist. Bei den Zwischenstellungen handelt es sich in einer Ausführungsform um Stellungen des inneren Bauteils, die von dem inneren Bauteil relative zu dem äußeren Bauteil eingenommen werden können, in denen der Hohlraum aber nicht mit einem an dem äußeren Bauteil gebildeten Anschluss in Fluidverbindung steht. Insoweit kann in den Zwischenstellungen weder Fluid über einen Anschluss in den Hohlraum einströmen, noch diesen verlassen. In einer Ausführungsform kann dieses zweckmäßig dadurch erreicht werden, dass eine den Zugang zum Hohlraum liefernde Öffnung in den Zwischenstellungen stets von einem an dem äußeren Bauteil gebildeten Oberflächenabschnitt verschlossen ist. Zweckmäßig berühren sich in einer Weiterbildung hierbei die Öffnung umgebende Oberflächenabschnitte des inneren Bauteil mit zugeordneten Oberflächenabschnitten des äußeren Bauteils, wodurch die Abdichtung des Hohlraums weiter optimiert.

Bei einer zweckmäßigen Ausgestaltung der Erfindung kann vorgesehen sein, dass der Hohlraum in der zweiten Stellung weiterhin in Fluidverbindung mit einem zweiten zugeordneten Anschluss steht, welcher dem zweiten Anschluss zugeordnet ist, wodurch über den Hohlraum eine bidirektionale Fluidverbindung zwischen dem zweiten Anschluss und dem zweiten zugeordneten Anschluss gebildet ist. Auf diese Weise ist es beispielsweise ermöglicht, über den zweiten zugeordneten Anschluss den Hohlraum mit Druck zu beaufschlagen, welcher insbesondere zum Ausdrücken der Körperflüssigkeitsprobe aus dem Hohlraum über den zweiten Anschluss dienen kann. Beispielsweise dient die Druckbeaufschlagung hierbei dazu, die Fluidprobe dem Analysegerät zuzuführen.

Eine weitere vorteilhafte Ausführungsform der Erfindung sieht vor, das das innere Bauteil in eine dritte Stellung verlagerbar ist, bedarfsweise mittels einer Drehung, in welcher der Hohlraum in Fluidverbindung mit einem dritten Anschluss steht, welcher konfiguriert ist, ein Funktionsfluid in den Hohlraum zuzuführen, und getrennt von dem ersten Anschluss und dem zweiten Anschluss gebildet ist. Als Funktionsfluid können beispielsweise ein Reinigungsfluid, ein Spülfluid, ein Desinfektionsfluid oder ein Kalibrierungsfluid zum Einsatz kommen. Getrennt von dem ersten und dem zweiten Anschluss ist über den dritten Anschluss beispielsweise ein Reinigen und Desinfizieren des Hohlraums ermöglicht. In Verbindung mit der Ausgestaltung des dritten Abschlusses bestehen die in Verbindung mit dem ersten und dem zweiten Anschluss beschriebenen Variationsmöglichkeiten entsprechend. Zweckmäßig ist der Hohlraum in der dritten Stellung getrennt von dem ersten Anschluss, dem zweiten Anschluss sowie dem ersten zugeordneten und dem zweiten zugeordneten Anschluss.

Eine bevorzugte Weiterbildung der Erfindung sieht vor, dass der Hohlraum in der dritten Stellung in Fluidverbindung mit einem dritten zugeordneten Anschluss steht, welcher dem dritten Anschluss zugeordnet ist, wodurch über den Hohlraum eine bidirektionale Fluidverbindung zwischen dem dritten Anschluss und dem dritten zugeordneten Anschluss gebildet ist. Auf diese Weise ist eine durchgehende Fluidverbindung zwischen dem dritten Anschluss und dem dritten zugeordneten Anschluss geschaffen, so dass der Hohlraum beispielsweise von dem Funktionsfluid in einer Flussrichtung oder einer entgegengesetzten Flussrichtung durchströmt werden kann.

Für Ausgestaltungen des Verfahrens zum Betreiben der Verteilvorrichtung für eine Körperflüssigkeitsprobe in einem Körperflüssigkeitsentnahme- und Infusionssystem nach den abhängigen Unteransprüchen gelten die in Verbindung zughörigen Merkmalen vorangehend gemachten Erläuterungen entsprechend. Je nach Anwendungsfall können darüber hinausgehende Verfahrensgestaltungen bei der Nutzung der Verteilvorrichtung und der wahlweise angeschlossenen Geräte und Leitungssystem vorgesehen sein, welche sich die konstruktiven Ausgestaltungsvarianten der und Anschlussmöglichkeiten für die Verteilvorrichtung zu Nutze machen.

### Beschreibung bevorzugter Ausführungsbeispiele der Erfindung

Die Erfindung wird im Folgenden an Hand von Ausfuhrungsbeispielen unter Bezugnahme auf Figuren einer Zeichnung näher erläutert. Hierbei zeigen:
- Fig. 1: eine schematische Darstellung einer Verteilvorrichtung für eine Körperflüssigkeits- probe, wobei eine Fluidverbindung zwischen einem ersten Anschluss und einem er- sten zugeordneten Anschluss gebildet ist,
- Fig. 2: eine schematische Darstellung der Verteilvorrichtung aus Fig. 1, wobei eine Fluid- verbindung zwischen einem zweiten Anschluss und einem zweiten zugeordneten An- schluss gebildet ist,
- Fig. 3: eine schematische Darstellung der Verteilvorrichtung aus Fig. 2, wobei eine Körper- flüssigkeitsprobe über den zweiten Anschluss abgegeben wird,
- Fig. 4: eine schematische Darstellung der Verteilvorrichtung aus Fig. 1, wobei zwischen ei- nem Dritten Anschluss und einem dritten zugeordneten Anschluss eine Fluidverbin- dung gebildet ist,
- Fig. 5: eine schematische Darstellung der Verteilvorrichtung aus Fig. 4, wobei eine Infusi- onslösung eingebracht ist,
- Fig. 6: eine schematische Darstellung der Verteilvorrichtung aus Fig. 5, wobei nun eine Flu- idverbindung zwischen dem ersten Anschluss und dem zugeordneten ersten An- schluss gebildet ist,
- Fig. 7: eine schematische Darstellung der Verteilvorrichtung aus Fig. 6, wobei eine Infusion fortgesetzt wird,
- Fig. 8: eine schematische Darstellung der Verteilvorrichtung für eine Körperflüssigkeitspro- be nach Fig. 1, wobei eine verminderte Anzahl von Anschlüsse gebildet ist, und
- Fig. 9: eine schematische Darstellung der Verteilvorrichtung für eine Körperflüssigkeitspro- be nach Fig. 1, wobei zusätzliche, einander zugeordnete Anschlüsse gebildet sind.

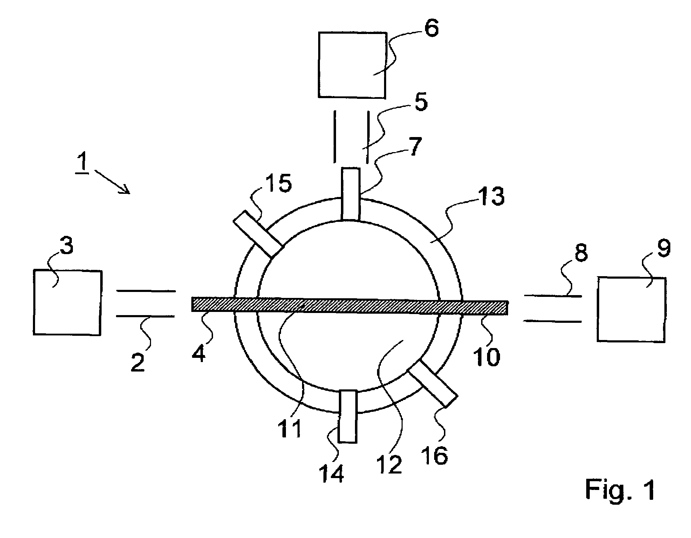

Im Folgenden wird eine Verteilvorrichtung für eine Körperflüssigkeitsprobe in einem Körperflüssigkeitsentnahme- und Infusionssystem unter Bezugnahme auf die Fig. 1 bis 7 näher erläutert. Hierbei werden für gleiche Merkmale dieselben Bezugszeichen verwendet.

Fig. 1 zeigt eine schematische Darstellung einer Verteilvorrichtung 1, mit welcher eine Körperflüssigkeit, insbesondere Blut, in einem System mit mehreren Fluidleitungen verteilt werden kann. Gemäß der schematischen Darstellung in Fig. 1 ist die Verteilvorrichtung 1 patientenseitig über ein patientenseitiges Leitungssystem 2 mit einem Patienten 3 verbunden. Das patientenseitige Leitungssystem 2 ist mit einem ersten Anschluss 4 verbunden. Analysatorseitig ist die Verteilvorrichtung 1 über ein analysatorseitiges Leitungssystem 5 mit einem Analysegerät 6 verbunden, welches eingerichtet ist, ein über die Verteilvorrichtung 1 an das Analysegerät 6 abgegebenes Fluid zu analysieren, beispielsweise eine Blutprobe. Das analysatorseitige Leitungssystem 5 ist an einen zweiten Anschluss 7 gekoppelt. Weiterhin ist die Verteilvorrichtung 1 gemäß Fig. 1 infusionsseitig über ein infusionsseitiges Leitungssystem 8 an eine Infusionseinrichtung 9 gekoppelt. Auf diese Weise kann über das infusionsseitige Leitungssystem 8, welches an einen ersten zugeordneten Anschluss 10 angeschlossen ist, eine Infusionslösung durch einen Hohlraum 11, der seinerseits in einem inneren Bauteil 12 gebildet ist, zu dem patientenseitigen Leitungssystem 2 geführt werden. Bei umgekehrter Strömungsrichtung gelangt über den ersten Anschluss 4 eine Körperflüssigkeit des Patienten 3 in den Hohlraum 11, beispielsweise Blut.

Gemäß Fig. 1 sind an einem ein Gehäuse bildenden, äußeren Bauteil 13, in welchem das innere Bauteil 12 mit dem Hohlraum 11 drehbar gelagert ist, weiterhin ein zweiter zugeordneter Anschluss 14 sowie ein dritter Anschluss 15 und ein dritter zugeordneter Anschluss 16 gebildet. Einem oder mehreren der Anschlüsse, beispielsweise dem zweiten zugeordneten Anschluss 14 oder dem dritten Anschluss 15, können geeignete Ventile vorgeschaltet sein, insbesondere zur Ausbildung von getrennten Kreisläufen für unterschiedliche Funktionsfluide.

Das äußere Bauteil 13 und das innere Bauteil 12 sind beispielsweise als Spritzgussbauteile aus einem Kunststoff ausgeführt. Das innere Bauteil 12 ist passgenau in das äußere Bauteil 13 eingelassen. Mittels Drehen kann das innere Bauteil 12 unter Überwindung der Reibungskräfte zwischen zugeordneten Oberflächen von innerem und äußerem Bauteil 12, 13 in verschiedene Drehstellungen gebracht werden, in denen der Hohlraum 11 bei der in Fig. 1 dargestellten Ausführungsform mit jeweiligen Anschlüssen in Fluidverbindung steht. In Zwischenstellung (nicht dargestellt) ist der Hohlraum 11 so positioniert, dass keine Fluidverbindung zu einem der Anschlüsse besteht.

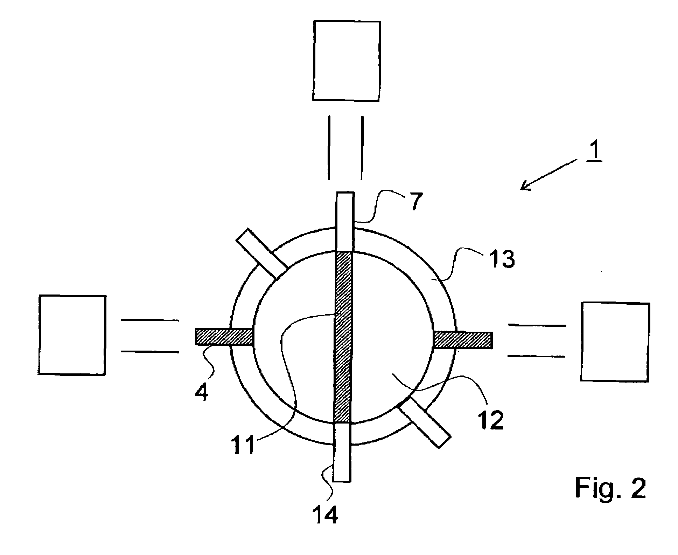

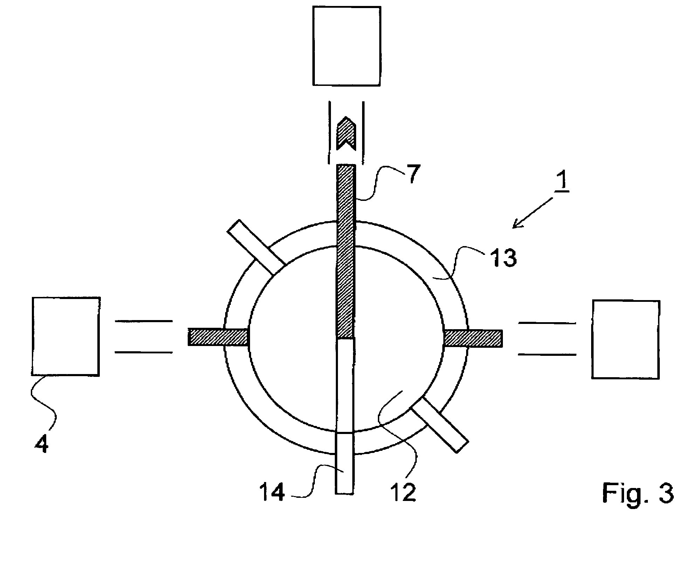

Fig. 2 zeigt eine schematische Darstellung der Verteilvorrichtung 1 aus Fig. 1, wobei das innere Bauteil 12 mittels Drehen in eine Verlagerungsstellung gebracht ist, in welcher eine Fluidverbindung zwischen dem zweiten Anschluss 7 und dem zweiten zugeordneten Anschluss 14 gebildet ist. Nachdem in der in Fig. 1 gezeigten Verlagerungsstellung des inneren Bauteils 12 eine Blutprobe in den Hohlraum 11 eingeströmt ist, kann diese nun über den zweiten Anschluss 7 dem analysatorseitigen Leitungssystem 5 und schließlich dem Analysegerät 6 zugeführt werden, insbesondere mittels Druckbeaufschlagung über den zweiten zugeordneten Anschluss 14, was in Fig. 3 schematisch gezeigt ist.

Unter Ausnutzung der Fig. 2 dargestellten Stellung des Hohlraums 11 oder einer anderen Stellung, welche den Hohlraum 11 von dem ersten Anschluss 4 trennt, können Funktionsflüssigkeiten in das Analysegerät 6 eingebracht werden, zum Beispiel eine Kalibricrflüssigkeit zum Kalibrieren oder eine Kontrollflüssigkeit.

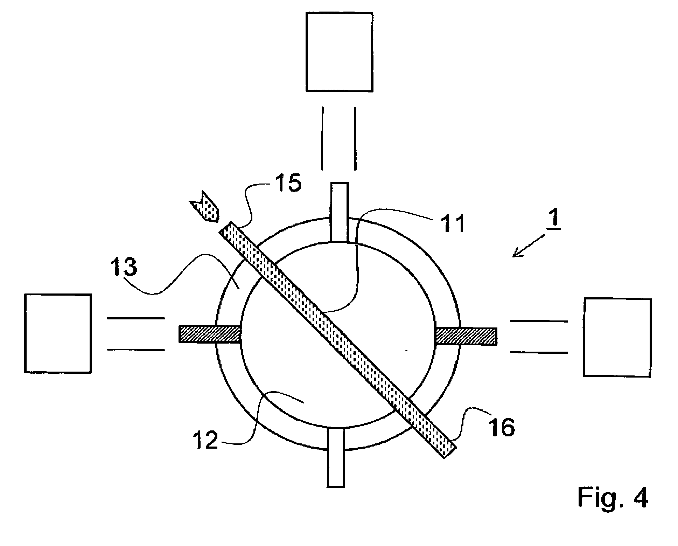

Fig. 4 zeigt eine schematische Darstellung der Verteilvorrichtung 1 aus Fig. 1, wobei nun das innere Bauteil 12 in eine Verlagerungsstellung gebracht ist, in welcher über den Hohlraum 11 eine Fluidverbindung zwischen dem dritten Anschluss 15 und dem dritten zugeordneten Anschluss 16 gebildet ist. In dieser Verlagerungsstellung befindet sich das innere Bauteil 12 in einer Reinigungsposition, in welcher über den dritten Anschluss15 ein Reinigungsfluid eingebracht werden kann. Auf diese Weise wird der Hohlraum 11 gewaschen. Weiterhin kann ein Desinfektionsmittel eingebracht werden. Ein Reinigungs- oder Waschflüssigkeit kann auch über den dritten Anschluss 15 eingebracht werden (vgl. Stellung des Hohlraums 11 in Fig. 2), wodurch eine Reinigung oder Spülung des analysatorseitigen Leitungssystems 5 realisiert werden kann.

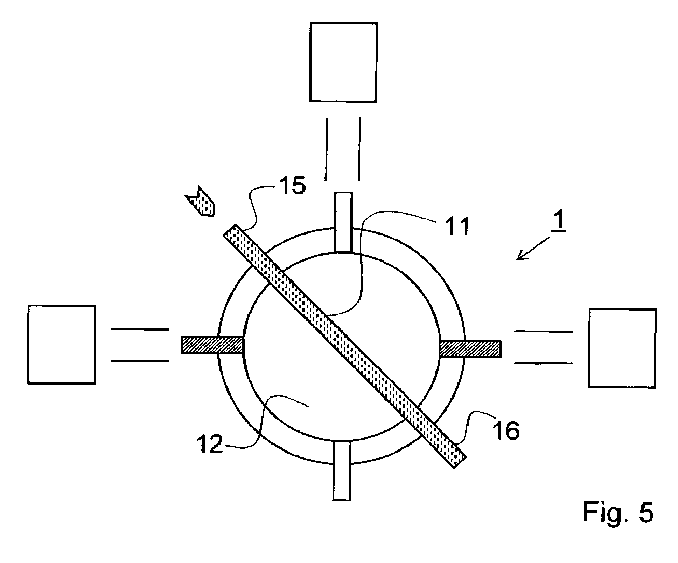

Schließlich wird, was in Fig. 5 schematisch gezeigt ist, der Hohlraum 11 mit Infusionslösung oder einer Kochsalzlösung ausgespült, um im Infusionskanal Luftblasen zu vermeiden. Hierbei kann das Einbringen der Infusionslösung auch dazu dienen, einen in dem Hohlraum 11 verbliebenen Rest der Körperflüssigkeit in den Körper des Patienten zurück zu spülen. Insoweit handelt es sich dann nicht um eine Infusionsflüssigkeit zu therapeutischen Zwecken, welche auch als Spülflüssigkeit bezeichnet werden kann.

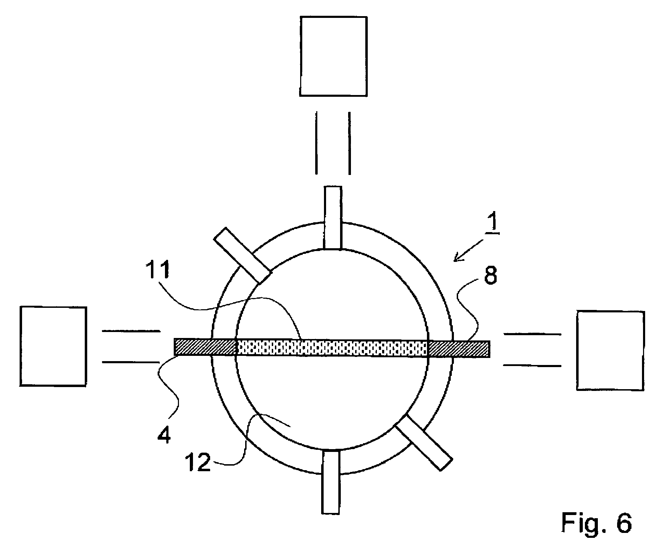

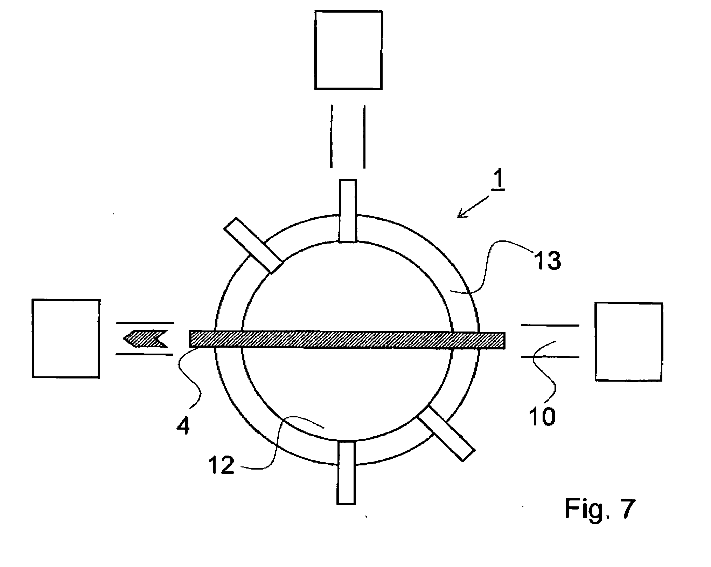

Fig. 6 zeigt dann schematisch, wie der Hohlraum 11, welcher mit der Infusionslösung oder der Kochsalzlösung gefüllt ist, nun wieder mit dem ersten Anschluss 4 und dem ersten zugeordneten Anschluss 8 in Fluidverbindung steht, um eine Infusion fortzusetzen, was dann in Fig. 7 schematisch gezeigt ist.

Die Verteilvorrichtung aus den Fig. 1 bis 7 kann in Anlehnung an die vorangehenden Ausführungen oder wahlweise abweichend hiervon gemäß der in Tabelle 1 schematisch aufgezeigten Art und Weise betrieben werden. Hierbei sind in der Spalte "Stellung" unter Verwendung der entsprechenden Bezugszeichen die mit dem Hohlraum 11 in Verbindung stehenden Anschlüsse genannt.

**Tabelle 1**

| Schritt | Stellung | Aktion |
|---|---|---|
| 1 | 4-11-10 | Infusionsflüssigkeit fließt über eine Leitungsstrecke 8-10-11-4-2 zum Patienten |
| 2 | 4-11-10 | Blut fließt über den ersten Anschluss 4 in den Hohlraum 11 |
| 3 | | Verlagerung des inneren Bauteils 12 in die Stellung 7-11-14 |
| 4 | 7-11-14 | Blut fließt vom Hohlraum 11 über den zweiten Anschluss 7 in das Analysatorgerät 6, wonach eine Messung erfolgt. Über den zweiten zugeordneten Anschluss 14 strömt ein Funktionsfluid nach, vorzugsweise Luft. |
| 5 | | Verlagerung des inneren Bauteils 12 in die Stellung 15-11-16 |
| 6 | 15-11-16 | Der Hohlraum 11 wird mittels einer Waschflüssigkeit gereinigt. Dabei strömt die Waschflüssigkeit über den dritten Anschluss 15 ein. Im Nachgang zu dem Waschvorgang wird die Waschflüssigkeit vorzugsweise durch Luft ersetzt. |
| 7 | | Weiter mit Schritt 8 oder mit Schritt 12 |
| 8 | 15-11-16 | Im Hohlraum 11 vorhandenes Fluid wird durch eine weitere Funktionsflüssigkeit ersetzt, beispielsweise eine Kalibrier- oder eine |
| | | QC-Flüssigkeit (Kontrollflüssigkeit). |
| 9 | | Verlagerung des inneren Bauteils 12 in die Stellung 7-11-14 |
| 10 | 7-11-14 | Im Hohlraum 11 befindliche weitere Funktionsflüssigkeit strömt über den zweiten Anschluss 7 und das analysatorseitige Leitungssystem 5 in das Analysatorgerät 6, wonach eine Kalibrier- oder eine QC-Messung erfolgt. |
| 11 | | Verlagerung des inneren Bauteils 12 in die Stellung 15-11-16 und weiter mit Schritt 8 oder mit Schritt 12 |
| 12 | 15-11-16 | Im Hohlraum 11 vorhandenes Fluid wird durch Infusionsflüssigkeit ersetzt. |
| 13 | | Verlagerung des inneren Bauteils 12 in die Stellung 4-11-10 |
| 14 | 4-11-10 | Infusionsflüssigkeit fließt über den ersten Anschluss 4 und das patientenseitige Leitungssystem 2 zum Patienten, wobei aus Schritt 2 in dem patientenseitigen Leitungssystem 2, dem ersten Anschluss 4, dem ersten zugeordneten Anschluss 10 und dem infusionsseitigen Leitungssystem 8 vorhandene Restflüssigkeit in den Körper zurückgespült wird. |
| 15 | 4-11-10 | weiter mit Schritt 1 |

Am dritten Anschluss 15 können ein oder mehrere Ventile angeschlossen sein, so dass wahlweise Luft oder eine von mehreren Funktionsflüssigkeiten eingebracht werden können. Optional handelt es sich bei der Waschflüssigkeit und der Infusionsflüssigkeit um ein und dieselbe Flüssigkeit. Zur Initialisierung (Inbetriebnahme) der erläuterten Anordnung mit der Verteilvorrichtung 1 sind üblicherweise hier nicht näher ausgeführte, dem Schritt 1 vorgelagerte Schritte vorgesehen. Zur Abkopplung der erläuterten Anordnung gibt es hier nicht näher ausgeführte, dem der Schritte 1 bis 15 nachgelagerte Schritte.

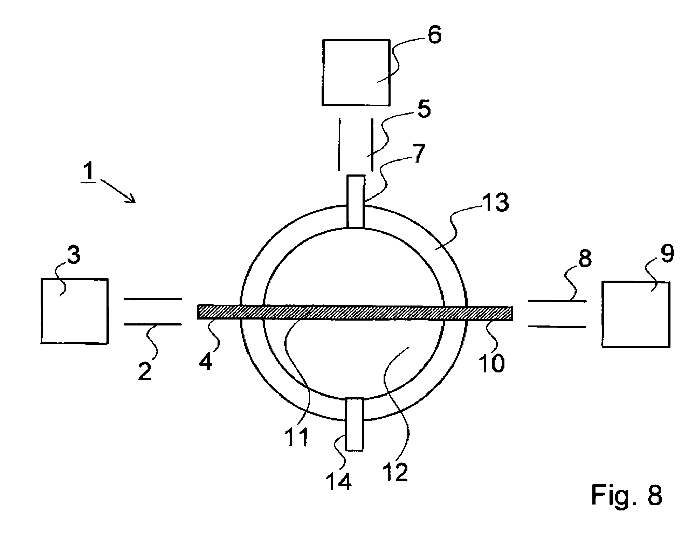

Fig. 8 zeigt eine schematische Darstellung der Verteilvorrichtung für eine Körperflüssigkeitsprobe nach Fig. 1, wobei eine verminderte Anzahl von Anschlüsse gebildet ist. Die Verteilvorrichtung aus Fig. 8 kann gemäß der in Tabelle 2 schematisch aufgezeigten Art und Weise betrieben werden. Hierbei sind wieder in der Spalte "Stellung" unter Verwendung der entsprechenden Bezugszeichen die mit dem Hohlraum 11 in Verbindung stehenden Anschlüsse genannt.

**Tabelle 2**

| Schritt | Stellung | Aktion |
|---|---|---|
| 1 | 4-11-10 | Infusionsflüssigkeit fließt über eine Leitungsstrecke 8-10-11-4-2 zum Patienten |
| 2 | 4-11-10 | Blut fließt über den ersten Anschluss 4 in den Hohlraum 11 |
| 3 | | Verlagerung des inneren Bauteils 12 in die Stellung 7-11-14 |
| 4 | 7-11-14 | Blut fließt vom Hohlraum 11 über den zweiten Anschluss 7 in das Analysegerät 6, wonach eine Messung erfolgt. Über den zweiten zugeordneten Anschluss 14 strömt ein Funktionsfluid nach, vorzugsweise Luft. |
| 5 | 7-11-14 | Eine Leitungsstrecke 14-11-7-5 wird mit einer Waschflüssigkeit gereinigt. Hierbei strömt die Waschflüssigkeit wird über den zweiten zugeordneten Anschluss 14 oder über das analysatorseitige Leitungssystem 5 ein. Im Nachgang zu dem Waschvorgang wird die Waschflüssigkeit ersetzt, vorzugsweise durch Luft. |
| 6 (optio nal) | 7-11-14 | Eine oder mehrere weitere Funktionsflüssigkeiten werden sequenziell über den zweiten zugeordneten Anschluss 14 eingebracht, gegebenenfalls durch Luftpakete getrennt, und strömen über eine Leitungsstrecke 14-11-7 das Analysegerät 6, wonach Kalibrier- bzw. QC-Messungen erfolgen. |
| 7 | 7-11-14 | Aus den Schritten 5 oder 6 vorhandene Funktionsflüssigkeit oder Luft wird durch Infusionsflüssigkeit ersetzt. |
| 8 | | Verlagerung des inneren Bauteils 12 in die Stellung 4-11-10 |
| 9 | 4-11-10 | Infusionsflüssigkeit fließt zum Patienten, wobei aus Schritt 2 in dem patientenseitigen Leitungssystem 2, dem ersten Anschluss 4, dem ersten zugeordneten Anschluss 10 und dem infusionsseitigen Leitungssystem 8 vorhandene Restflüssigkeit in den Körper zurückgespült wird. |
| 10 | 4-11-10 | weiter mit Schritt 1 |

Am zweiten zugeordneten Anschluss 14 können ein oder mehrere Ventile angeschlossen sein, so dass wahlweise Luft oder eine von mehreren Funktionsflüssigkeiten eingebracht werden können. Optional handelt es sich bei der Waschflüssigkeit und Infusionsflüssigkeit um ein und dieselbe Flüssigkeit. Zur Initialisierung (Inbetriebnahme) der erläuterten Anordnung mit der Verteilvorrichtung 1 gibt es hier nicht näher ausgeführte, dem Schritt 1 vorgelagerte Schritte. Zur Abkopplung der erläuterten Anordnung können hier nicht näher ausgeführte, einem der Schritte 1 bis 10 nachgelagerte Schritte vorgesehen sein.

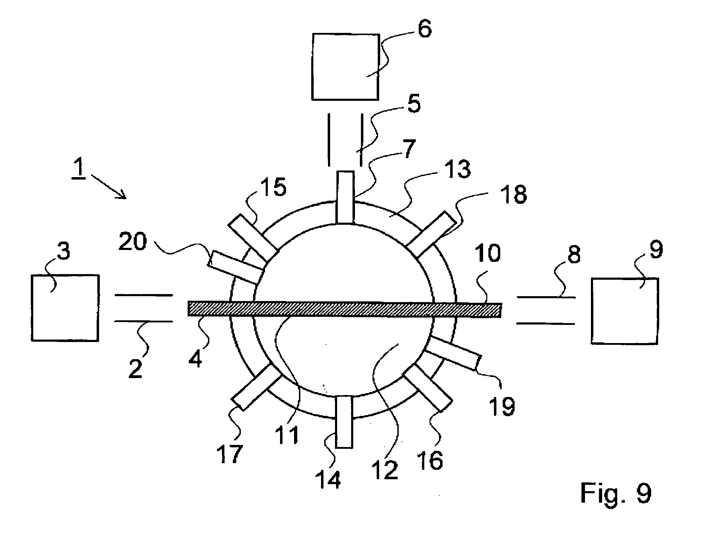

Fig. 9 zeigt eine schematische Darstellung der Verteilvorrichtung für eine Körperflüssigkeitsprobe nach Fig. 1, wobei zusätzliche, einander zugeordnete Anschlüsse 17, 18 und 19, 20 gebildet sind. Unter Verwendung der zusätzlichen, einander zugeordneten Anschlüsse 17, 18 und 19, 20 können weitere separate Kreisläufe realisiert werden.

Die vorangehend beschriebenen Betriebszyklen der Verteilvorrichtung in ihren verschiedenen Ausgestaltungen bilden bevorzugte Ausgestaltungen, die jedoch anderen Anforderungen im konkreten Anwendungsfall geändert und angepasst werden können.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Verteilvorrichtung (1) für eine Körperflüssigkeitsprobe, insbesondere eine Blutprobe, mit einem äußeren Bauteil (13), an welchem mehrere Anschlüsse gebildet sind, und einem inneren Bauteil (12), welches wenigstens teilweise in einer Aufnahme in dem äußeren Bauteil (13) angeordnet ist, zwischen Verlagerungsstellungen verlagerbar ist und einen zum Aufnehmen einer Fluidprobe konfigurierten Hohlraum (11) aufweist, wobei:
- in einer ersten Stellung des inneren Bauteils (12) über den Hohlraum (11) zwischen einem ersten Anschluss (4) und einem ersten zugeordneten Anschluss (10), welcher dem ersten Anschluss (4) zugeordnet ist, eine bidirektionale Fluidverbindung gebildet ist und der Hohlraum (11) getrennt von einem zweiten Anschluss (7) ist, und
- in einer zweiten Stellung der Hohlraum (11) von dem ersten Anschluss (4) und dem ersten zugeordneten Anschluss (10) getrennt ist und in Fluidverbindung mit dem zweiten Anschluss (7) steht.

2. Vorrichtung nach Anspruch 1, **dadurch gekennzeichnet, dass** mit dem Hohlraum (11) eine Kanalstruktur in dem inneren Bauteil (12) gebildet ist.

3. Vorrichtung nach Anspruch 1 oder 2, **dadurch gekennzeichnet, dass** das innere Bauteil (12) mittels einer Drehbewegung zwischen der ersten Stellung und der zweiten Stellung verlagerbar ist.

4. Vorrichtung nach mindestens einem der vorangehenden Ansprüche, **dadurch gekennzeichnet, dass** das innere Bauteil (12) in die Aufnahme formschlüssig eingepasst ist.

5. Vorrichtung nach mindestens einem der vorangehenden Ansprüche, **dadurch gekennzeichnet, dass** die erste Stellung und die zweite Stellung benachbarte Verlagerungsstellungen des inneren Bauteils (12) sind.

6. Vorrichtung nach mindestens einem der vorangehenden Ansprüche, **dadurch gekennzeichnet, dass** der Hohlraum (11) in Zwischenstellungen, welche von der ersten Stellung und der zweiten Stellung verschieden sind, als anschlussfreier Hohlraum gebildet ist.

7. Vorrichtung nach mindestens einem der vorangehenden Ansprüche, **dadurch gekennzeichnet, dass** der Hohlraum (11) in der zweiten Stellung in Fluidverbindung mit einem zweiten zugeordneten Anschluss (14) steht, welcher dem zweiten Anschluss (14) zugeordnet ist, wodurch über den Hohlraum (11) eine bidirektionale Fluidverbindung zwischen dem zweiten Anschluss (7) und dem zweiten zugeordneten Anschluss (14) gebildet ist.

8. Vorrichtung nach mindestens einem der vorangehenden Ansprüche, **dadurch gekennzeichnet, dass** das innere Bauteil (12) in eine dritte Stellung verlagerbar ist, in welcher der Hohlraum (11) in Fluidverbindung mit einem dritten Anschluss (15) steht, welcher konfiguriert ist, ein Funktionsfluid in den Hohlraum (11) zuzuführen, und getrennt von dem ersten Anschluss (4) und dem zweiten Anschluss (7) gebildet ist.

9. Vorrichtung nach Anspruch 8, **dadurch gekennzeichnet, dass** der Hohlraum (11) in der anderen Stellung in Fluidverbindung mit einem dritten zugeordneten Anschluss (16) steht, welcher dem dritten Anschluss (15) zugeordnet ist, wodurch über den Hohlraum (11) eine bidirektionale Fluidverbindung zwischen dem dritten Anschluss (15) und dem dritten zugeordneten Anschluss (16) gebildet ist.

10. Körperflüssigkeitsentnahme- und Infusionssystem, insbesondere Blutentnahme- und Infusionssystem, mit einer Probenverteilvorrichtung (1) nach mindestens einem der vorangehenden Ansprüche, wobei der erste Anschluss (4) als ein patientenseitiger Anschluss, der erste zugeordnete Anschluss (10) als ein infusionsseitiger Anschluss und der zweite Anschluss (7) als ein analysatorseitiger Anschluss gebildet sind.

11. Körperflüssigkeitsanalysevorrichtung, insbesondere Blutanalysevorrichtung, mit einer integrierten Verteilvorrichtung (1) nach mindestens einem der Ansprüche 1 bis 9, wobei der erste Anschluss (4) als ein patientenseitiger Anschluss, der erste zugeordnete Anschluss (10) als ein infusionsseitiger Anschluss und der zweite Anschluss (7) als ein analysatorseitiger Anschluss gebildet sind.

12. Verfahren zum Betreiben einer Verteilvorrichtung (1) für eine Körperflüssigkeitsprobe in einem Körperflüssigkeitsentnahme- und Infusionssystem, insbesondere einem Blutentnahme- und Infusionssystem, mit einem äußeren Bauteil (13), an welchem mehrere Anschlüsse gebildet sind, und einem inneren Bauteil (12), welches wenigstens teilweise in einer Aufnahme in dem äußeren Bauteil (13) angeordnet ist, zwischen Verlagerungsstellungen verlagerbar ist und einen zum Aufnehmen einer Körperflüssigkeitsprobe konfigurierten Hohlraum (11) aufweist, wobei das Verfahren die folgenden Schritte aufweist:
- Anordnen des inneren Bauteils (13) in einer ersten Stellung, in welcher über den Hohlraum (11) zwischen einem ersten Anschluss (4) und einem ersten zugeordneten Anschluss (10), welcher dem ersten Anschluss (4) zugeordnet ist, eine bidirektionale Fluidverbindung gebildet und der Hohlraum (11) getrennt von einem zweiten Anschluss (7) ist,
- Einbringen einer Körperflüssigkeitsprobe über den ersten Anschluss in den Hohlraum (11),
- Verlagern des inneren Bauteils (13) in eine zweite Stellung, in welcher der Hohlraum (11) von dem ersten Anschluss (4) und dem ersten zugeordneten Anschluss (10) getrennt ist und in Fluidverbindung mit dem zweiten Anschluss (7) steht,
- wenigstens teilweises Abgeben der Körperflüssigkeitsprobe aus dem inneren Hohlraum (11) über den zweiten Anschluss (7),
- Verlagern des inneren Bauteils (13) in die erste Stellung und
- Bereitstellen einer Infusionsflüssigkeit an dem ersten zugeordneten Anschluss (10) für eine Abgabe über den Hohlraum (11) und den ersten Anschluss (4).

13. Verfahren nach Anspruch 12, **dadurch gekennzeichnet, dass** das innere Bauteil (13) in eine dritte Stellung verlagert wird, in welcher der Hohlraum (11) in Fluidverbindung mit einem dritten Anschluss (15) steht, der getrennt von dem ersten Anschluss (4) und dem zweiten Anschluss (7) gebildet ist, und dass ein Funktionsfluid in den Hohlraum (11) zugefühurt wird.

14. Verfahren nach Anspruch 13, **dadurch gekennzeichnet, dass** mittels des Zuführens des Funktionsfluids der Hohlraum (11) gespült wird.

15. Verfahren nach mindestens einem der Ansprüche 12 bis 14, **dadurch gekennzeichnet, dass** beim Einbringen der Körperflüssigkeitsprobe über den ersten Anschluss in den Hohlraum (11) eine entnommene Blutprobe eingebracht wird.

16. Verfahren nach mindestens einem der Ansprüche 12 bis 15, **dadurch gekennzeichnet, dass** beim wenigstens teilweisen Abgeben der Körperflüssigkeitsprobe aus dem inneren Hohlraum (11) über den zweiten Anschluss (7) eine abgegebene Menge der Körperflüssigkeitsprobe an eine Analyseeinrichtung abgegeben wird.
